# EUROPÄISCHE PATENTANMELDUNG

(11) **EP 1 179 353 A1**
(43) Veröffentlichungstag der Anmeldung: **13.02.2002**
(21) Anmeldenummer: 00117191.7
(22) Anmeldetag: 11.08.2000
(51) Int. Cl.: A61L 27/34, A61L 33/06

(54) **Antithrombogene Implantate mit Beschichtung aus Polyphosphazenen und einem pharmakologisch aktiven Wirkstoff**

(71) Anmelder: B. BRAUN MELSUNGEN AG, 34212 Melsungen (DE)
(72) Erfinder: Nagel, Stefan, 13503 Berlin (DE); Boxberger, Michael, 12103 Berlin (DE)
(74) Vertreter: Helbing, Jörg, Dr. Dipl.-Chem.

(57) **Zusammenfassung**

Die vorliegende Erfindung betrifft künstliche Implantate mit einem biokompatiblen Überzug aus einem Polyphosphazen, der antithrombogene Eigenschaften aufweist und weiterhin einen pharmakologisch aktiven Wirkstoff enthält, sowie Verfahren zu deren Herstellung.
Das Polyphosphazen ist vorzugsweise Poly[bis(trifluoroethoxy)phosphazen].
In einer erfindungsgemäßen Ausführungsform ist zwischen der Oberfläche des Substrats und dem biokompatiblen Überzug eine Schicht angeordnet, die einen Adhäsionspromotor enthält. Hierbei werden vorzugsweise aminoterminierte Silane, auf einem Aminosilan basierende Verbindungen oder Alkylphosphonsäuren verwendet. Ein besonders bevorzugter Adhäsionspromotor ist Aminopropyltrimethoxysilan.

## Beschreibung

Die vorliegende Erfindung betrifft künstliche Implantate mit einem biokompatiblen Überzug, der antithrombogenen Eigenschaften aufweist, und weiterhin einen pharmakologisch aktiven Wirkstoff enthält, sowie Verfahren zu deren Herstellung.

Die größten Komplikationen, die durch künstliche Implantate auftreten, sind in der vermehrten Thrombozytenablagerung auf der körperfremden Oberfläche zu sehen. Eine derartige Thrombenbildung beim Kontakt von menschlichem Blut mit der körperfremden Oberfläche, beispielsweise künstliche Herzklappen, ist im Stand der Technik beschrieben (vgl. Informationsmaterial der Firma Metronic Hall, Bad Homburg, Carmeda BioAktive Oberfläche (CBSA), Seite 1-21, B. D. Ratner, "The blood compatibility catastrophe", J. of Biomed. Mat. Res., Vol. 27, 283-287 sowie C. W. Akins, "Mechanical Cardiac Valvular Prostheses", The Society of Thoracic Surgeons, 161-171 (1991)). Beispielsweise bestehen die auf dem Weltmarkt befindlichen künstlichen Herzklappen aus pyrolisiertem Kohlenstoff und zeigen eine erhöhte Neigung zur Ausbildung von Thromben (vgl. C. W. Akins, s. o.).

In der DE-C-19613048 wurde die polymere Verbindung Poly[bis(trifluoroethoxy)phosphazen], deren gute antithrombogene Wirkung aus Holleman Wiberg, "Stickstoffverbindungen des Phosphors" Lehrbuch der anorganischen Chemie, 666-669, 91.-100. Auflage, Walter de Gruyter Verlag (1985) sowie Tur, Vinogradova, u. a. "Entwicklungstendenzen bei Polymeranalogen Umsetzungen von Polyphosphazen", Acta Polymerica 39, 424-429, Nr. 8 (1988) bekannt war, zur Beschichtung von künstlichen Implantaten eingesetzt. Im Einzelnen beschreibt die DE-C-19613048 ein künstliches Implantat, umfassend ein Implantatmaterial als Substrat und einen mindestens teilweise auf die Oberfläche des Substrats aufgebrachten, biokompatiblen Überzug, der ein antithrombogenes Polymer mit der folgenden allgemeinen Formel (I) enthält: 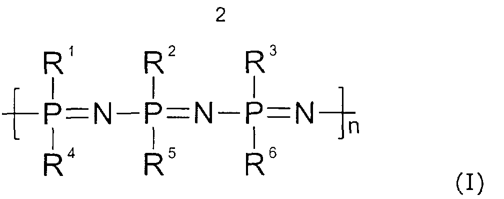 wobei R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeutet; sowie Herstellungsverfahren für solche künstlichen Implantate.

Ein Problem bei Implantaten wie Herzklappen und Stents (siehe DE-A-197 53 123) ist - unabhängig davon ob eine Beschichtung des Implantats mit dem vorliegenden antithrombogenen Material vorliegt - deren Restenoseneigung, d. h., eine Einengung durch Proliferation von glatten Muskelzellen in der Gefäßwand als biologische Antwort auf das Implantat. In einem Übersichtsartikel von Swanson und Gershlick (Stent, Vol. 2, 66 - 73 (1999)) werden eine Vielzahl von Ansätzen zum Aufbringen von geeigneten aktiven Wirkstoffen auf die Implantate erwähnt. U. a. wird auf S. 68 vorgeschlagen, polymerbeschichtete Stents einzusetzen, wobei das Polymer as Reservoir für Wirkstoffe dienen kann. Es wird jedoch sofort davon abgeraten, diesen Ansatz zu verfolgen, da in einer Teststudie, bei der Stents mit verschiedenen bioabbaubaren Polymeren, die ansonsten alle als biocompatibel *in vitro* bekannt waren, beschichtet wurden, eine erhöhte Entzündungsneigung *in vivo* gefunden wurde. Weiterhin beschreiben die US-Patente 5,788,979 und 5,980,972 das Beschichten von Materialien mit bioabbaubaren Polymeren, wobei die Beschichtung zusätzlich pharmakologisch aktive Substanzen enthalten kann.

In der WO 99/16477 ist ein alternativer Ansatz zur Verhinderung von übermäßiger Zellproliferation und Narbenbildung beschrieben worden. Hier wird ein raidioaktiv markiertes Polymer der vorstehend gezeigten Formel (I), bevorzugt ein Polymer, in dem ein radioaktives Phosphorisotop vorliegt, auf das Implantat aufgebracht. Die emittierte radioaktive Strahlung (bei ³²P β-Strahlung) soll unkontrolliertes Zellwachstum, das z. B. bei der Stentimplantation zur Restenose führt, verhindern. Allerdings sind bei dem Einsatz radioaktiver Materialien Sicherheitsauflagen und Nebenwirkungen zu berücksichtigen, die den unkomplizierten Einsatz solcher Implantate entgegenstehen.

Somit liegt der vorliegenden Erfindung die Aufgabe zugrunde, künstliche Implantate mit sowohl ausgezeichneten mechanischen Eigenschaften als auch mit antithrombogenen und -restenosen Eigenschaften bereitzustellen, um dadurch die Biokompatibilität und Verträglichkeit derartiger Implantate zu verbessern. Ferner sollen Verfahren zur Herstellung derartiger Implantate bereitgestellt werden.

Überraschenderweise wurde gefunden, dass das vorstehend definierte Polymer der Formel (I) ausgezeichnete Matrixeigenschaften für pharmakologisch aktive Wirkstoffe aufweist und diese Wirkstoffe, wenn auf ein Implantatmaterial aufgebracht, kontrolliert an seine Umgebung abgibt. Überraschenderweise wurde auch festgestellt, dass beim biologischen Abbau des Polymers der Formel (I) keine entzündliche Reaktion auftritt. Somit wird eine kontrollierte Wirkstoffabgabe nicht nur durch Diffusions- und Lösungsvorgänge, sondern auch durch den biologischen Abbau der Matrix und die damit verbundene Freisetzung von eingebundenen Wirkstoffen ermöglicht, ohne dass eine unerwünschte Entzündungsreaktion auftritt.

Gegenstand der vorliegenden Erfindung ist somit ein künstliches Implantat, umfassend ein Implantatmaterial als Substrat und einen mindestens teilweise auf die Oberfläche des Substrats aufgebrachten, biokompatiblen Überzug, der ein antithrombogenes Polymer mit der folgenden allgemeinen Formel (I) 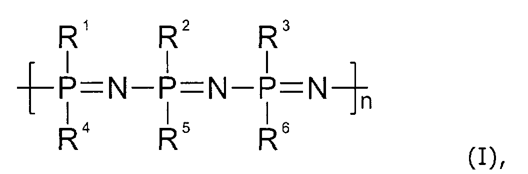 wobei R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeutet, und wenigstens einen weiteren (zusätzlichen) pharmakologisch aktiven Wirkstoff (nachfolgend kurz "Wirkstoff") umfasst.

In dem Polymer der Formel (I) ist vorzugsweise mindestens einer der Reste R¹ bis R⁶ ein Alkoxyrest, der mit mindestens einem Fluoratom substituiert ist.
In dem Polymer der Formel (I) sind die Alkylreste in den Alkoxy-, Alkylsufonyl-, und Dialkylaminoresten beispielsweise gerad oder verzweigtkettige Alkylreste mit 1 bis 20 Kohlenstoffatomen, wobei die Alkylreste beispielsweise mit mindestens einem Hologenatom, wie ein Fluoratom, substituiert sein können.

Beispiele für Alkoxyreste sind Methoxy-, Ethoxy-, Propoxy- und Butoxygruppen, die vorzugsweise mit mindestens einem Fluoratom substituiert sein können. Besonders bevorzugt ist die 2,2,2-Trifluoroethoxygruppe.

Beispiele für Alkylsulfonylreste sind Methyl-, Ethyl-, Propyl- und Butylsulfonylgruppen.

Beispiele für Dialkylaminoreste sind Dimethyl-, Diethyl-, Dipropyl- und Dibutylaminogruppen.

Der Arylrest im Aryloxyrest ist beispielsweise eine Verbindung mit einem oder mehreren aromatischen Ringsystemen, wobei der Arylrest beispielsweise mit mindestens einem, vorstehend definierten Alkylrest substituiert sein kann.

Beispiele für Aryloxyreste sind Phenoxy- und Naphthoxygruppen und Derivate davon.

Der Heterocycloalkylrest ist beispielsweise ein 3- bis 7-Atome enthaltend es Ringsystem, wobei mindestens ein Ringatom ein Stickstoffatom ist. Der Heterocycloalkylrest kann beispielsweise mit mindestens einem, vorstehend definierten Alkylrest substituiert sein kann. Beispiele für Heterocycloalkylreste sind Piperidinyl-, Piperazinyl-, Pyrrolidinyl- und Morpholinylgruppen und Derivate davon.

Der Heteroarylrest ist beispielsweise eine Verbindung mit einem oder mehreren aromatischen Ringsystemen, wobei mindestens ein Ringatom ein Stickstoffatom ist. Der Heteroarylrest kann beispielsweise mit mindestens einem, vorstehend definierten Alkylrest substituiert sein kann. Beispiele für Heteroarylreste sind Pyrrolyl-, Pyridinyl-, Pyridinolyl-, Isochinolinyl- und Chinolinylgruppen und Derivate davon.

In einer bevorzugten Ausführungsform der vorliegenden Erfindung enthält der biokompatible Überzug das antithrombogene Polymer Poly[bis(trifluoroethoxy)phosphazen].

Der Wirkstoff ist vorzugsweise ein antimitogener Wirkstoff, z. B. ein Zytostatikum (wie Paclitaxel usw.), ein PDGF-Hemmer (wie Tyrphostine usw.), ein Raf 1-Kinase-Hemmer, ein monoklonaler Antikörper zur Integrinblockade von Leukozyten, ein Antisensewirkstoff (wie Plasmid-DNA usw.), Superoxid-Dismutase, ein Radikalfänger (wie Probucol usw.), ein Steroid, ein Statin (wie Cerivastatin usw.), ein Corticosteroid (wie Methotrexat, Dexmethason, Methylprednisolan usw.), ein Adenylatcyclase-Inhibitor (wie Forskolin usw.), ein Somatostatinanalogon (wie Angiopeptin usw.), ein Antithrombinwirkstoff (wie Argatroban usw.), ein Stickstoffoxiddonator, ein Glycoprotein-IIb/IIIa-Rezeptorantagonist (wie Urokinasederivate, Abciximab, Tirofiban usw.), ein antithrombotisches Mittel (wie aktiviertes Protein C, PEG-Hirudin, Prostglandinanaloga usw.), ein vaskulärer endothelialer Wachstumsfaktor (VEGF), Trapidil usw. und Gemische derselben.

Für eine wirksame Verhinderung der Restenose ist es dabei empfehlenswert, dass ein möglichst hoher Wirkstoffgehalt in dem biokompatiblen Überzug vorliegt. Es konnte gezeigt werden, dass bis zu 50 Gew.-% des Überzugs aus Wirkstoff bestehen kann, ohne die mechanischen Eigenschaften desselben wesentlich zu beeinträchtigen. Der Anteil des Wirkstoffs an dem Überzug liegt erfindungsgemäß im Bereich von 0,01 bis 50 Gew.-%, vorzugsweise 0,2 bis 30 Gew.-%, was ungefähr einem Gewichtsverhältnis Polymer zu Wirkstoffen von 1:0,0001 bis 1:1, vorzugsweise 1:0,05 bis 1:0,5 entspricht.

Der biokompatible Überzug des erfindungsgemäßen künstlichen Implantats weist beispielsweise eine Dicke von 1 nm bis etwa 100 µm, vorzugsweise von 10 nm bis 10 µm und besonders bevorzugt bis etwa 1 µm auf.

Das als Substrat erfindungsgemäß verwendete Implantatmaterial weist keine besondere Beschränkung auf und kann jedes Implantatmaterial, wie Kunststoffe, Metalle, Metalllegierungen und Keramiken, sein. Beispielsweise kann das Implantatmaterial eine künstliche Herzklappe aus pyrolisiertem Kohlenstoff oder ein wie in der DE-A-197 53 123 beschriebener Stent sein.

In einer erfindungsgemäßen Ausführungsform des künstlichen Implantats ist zwischen der Oberfläche des Substrats und dem biokompatiblen Überzug eine Schicht angeordnet, die einen Adhäsionspromotor enthält.

Der Adhäsionspromotor bzw. Spacer ist beispielsweise eine siliciumorganische Verbindung, vorzugsweise ein aminoterminiertes Silan bzw. eine auf einem Aminosilan basierende Verbindung oder eine Alkylphosphonsäure. Besonders bevorzugt ist Aminopropyltrimethoxysilan.

Der Adhäsionspromotor verbessert insbesondere die Haftung des Überzugs auf der Oberfläche des Implantatmaterials durch Kopplung des Adhäsionspromotors an die Oberfläche des Implantatmaterials beispielsweise über ionische und/oder kovalente Bindungen und durch weitere Kopplung des Adhäsionspromotors an reaktive Bestandteile, insbesondere an das antithrombogene Polymer, des Überzugs beispielsweise über ionische und/oder kovalente Bindungen.

Weiterhin wird ein Verfahren zur Herstellung der erfindungsgemäßen künstlichen Implantate bereitgestellt, wobei der biokompatible Überzug durch Umsetzen des Substrats mit
(a) einem Gemisch aus dem antithrombogenen Polymer oder eine Vorstufe desselben und dem Wirkstoff oder
(b) dem antithrombogenen Polymer oder einer Vorstufe desselben unter Erhalt eines primären Polymerüberzugs und nachfolgendes Aufbringen/Eindringen des Wirkstoffs in den primären Polymerüberzug
auf das Substrat aufgebracht wird.

Besonders bevorzugt insbesondere für die Verfahrensvariante (a) ist - da der Wirkstoff oft empfindlich gegen drastische Reaktionsbedingungen ist, ein nasschemisches Verfahren. Hierbei wird das Substrat in eine Lösung, enthaltend das antithrombogene Polymer und Wirkstoff getaucht und ggf. nachfolgend das Lösungsmittel durch Erwärmen oder Anlegen von Vakuum entfernt. Dieser Vorgang wird so oft wiederholt, bis die gewünschte Schichtdicke der Beschichtung vorliegt.

Geeignete Lösungsmittel für diese Verfahren sind ausgewählt aus polaren aprotischen Lösungsmitteln wie Estern (wie Ethylacetat, Propylacetat, Butylacetat, Ethylpropionat, Ethylbutyrat usw.), Ketonen (wie Aceton, Ethylmethylketon usw.), Amiden (wie Dimethylformamid usw.), Sulfoxiden (wie DMSO usw.) und Sulfonen (sie Sulfolan usw.). Besonders bevorzugt ist Ethylacetat. Die Konzentration des Polymers in der Lösung ist 0,001 bis 0,5 M, vorzugsweise 0,01 bis 0,1 M, die Konzentration des Wirkstoffs richtet sich nach dem gewünschten Polymer/Wirkstoffverhältnis. Die Eintauchzeit liegt vorzugsweise im Bereich von 10 s bis 100 h. Die Tockenschritte werden im Vakuum, unter Luft oder Schutzgas im Temperaturintervall von beispielsweise etwa -20°C bis etwa 300 °C, bevorzugt 0°C bis 200°C und besonders bevorzugt von 20° C bis 100° C durchgeführt.

Bei stabilen Wirkstoffen können auch die anderen in der DE 196 13 048 erwähnten Verfahren wie das Verfahren des Aufbringens von Polydichlorphosphazen und nachfolgende Umsetzung mit reaktiven Verbindungen, des Aufschmelzens oder der Sublimation erfolgen. Diese Verfahren sind insbesondere für die erste Stufe der Verfahrensvariante (b) einsetzbar, in der das Aufbringen/Eindringen des Wirkstoffs in einem zweiten Schritt erfolgt, der dann vorzugsweise schonend nasschemisch, wie vorstehend beschrieben, erfolgen kann.

Bei dem Polydichlorphosphazen verwendenden Verfahren wird ein Gemisch aus Polydichlorphosphazen und Wirkstoff auf die Oberfläche des Substrats aufgebracht und mit mindestens einer reaktiven Verbindung, ausgewählt aus aliphatischen oder aromatischen Alkoholen oder deren Salze, Alkylsulfonen, Dialkylaminen und aliphatischen oder aromatischen Heterocyclen mit Stickstoff als Heteroatom, entsprechend der vorstehenden Definition von R¹ bis R⁶, umgesetzt. Das Polydichlorphosphazen wird dabei vorzugsweise unter Inertgasatmosphäre auf die Oberfläche des Substrats aufgebracht, gegebenenfalls an den Adhäsionspromotor gekoppelt und mit der reaktiven Verbindung umgesetzt. Alternativ kann Polydichlorphosphazen unter verringertem Druck oder unter Luftatmosphäre aufgebracht und gegebenenfalls an den Adhäsionspromotor gekoppelt werden.

Die Herstellung von Polymeren der Formel (I) wie z. B. von Poly[bis(trifluoroethoxy)phosphazen], ausgehend von Hexachlorcyclotriphosphazen, ist im Stand der Technik bekannt. Die Polymerisation von Hexachlorcyclotriphosphazen wird ausführlich in Korsak et al., Acta Polymerica 30, Heft 5, S. 245-248 (1979), beschrieben. Die Veresterung des durch die Polymerisation hergestelltes Polydichlorphosphazen wird in Fear, Thower und Veitch, J. Chem. Soc., S. 1324 (1958), beschrieben.

In einer bevorzugten Ausführungsform des erfindungsgemäßen Verfahrens wird vor dem Aufbringen des Gemisches aus Polymer oder Polymervorstufe und Wirkstoff bzw. vor dem Aufbringen von Polymer oder Polymervorstufe ein wie vorstehend definierter Adhäsionspromotor auf die Oberfläche des Substrat aufgebracht und beispielsweise über ionische und/oder kovalente Bindungen an die Oberfläche gekoppelt. Danach wird das antithrombogene Polymer z. B. von Polydichlorphosphazen auf die mit dem Adhäsionspromotor beschichtete Oberfläche des Substrats aufgebracht und beispielsweise über ionische und/oder kovatente Bindungen an den Adhäsionspromotor gekoppelt.

Der Adhäsionspromotor kann nasschemisch bzw. in Lösung oder aus der Schmelze oder durch Sublimation oder durch Aufsprühen auf das Substrat aufgebracht werden. Die nasschemische Ankopplung eines Adhäsionspromotors, basierend auf Aminosüanen an hydroxylierte Oberflächen, ist in Marco Mantar, Diplomarbeit, S. 23, Univ. Heidelberg (1991), beschrieben.

Vor dem Aufbringen von Polydichlorphosphazen, des Adhäsionspromotors oder des antithrombogenen Polymers kann die Oberfläche des Substrats z. B. mit Caroscher Säure oxidativ gereinigt werden. Die oxidative Reinigung von Oberflächen mit gleichzeitiger Hydroxylierung, wie sie beispielsweise für Implantate aus Kunststoffen, Metallen oder Keramik eingesetzt werden kann, ist in Ulman Abraham, Analysis of Surfare Properties, »An Introduction to Ultrahin Organic Films», 108,1991, beschrieben.

Zusammengefasst kann festgestellt werden, daß die erfindungsgemäßen künstlichen Implantate überraschenderweise die ausgezeichneten mechanischen Eigenschaften des Implantatmateriais als Substrat beibehalten und durch den erfindungsgemäß aufgebrachten Überzug, beispielsweise durch direkte Abscheidung aus der Lösung, nicht nur antithrombogene sondern auch antirestenose Eigenschaften aufweisen, was die Biokompatibilität und Einsetzbarkeit derartiger künstlicher Implantate drastisch verbessert. Diese überraschenden Ergebnisse können anhand von Röntgenphotoelektronen (XPS)-Spektren leicht nachgewiesen werden.

Die vorliegende Erfindung wird nachstehend durch Beispiele näher erläutert.

### Beispiele

### Beispiel 1

A: Die Herstellung des dem Poly[bis(trifluoroethoxy)phosphazen] zugrundeliegenden Polydichlorphosphazen erfolgt durch Polymerisation von Hexachlorcyclotriphosphazen in einer Ampulle mit einem Durchmesser von 5,0 mm bei 250 °C ± 1 °C sowie einem in der Ampulle herrschenden Druck von 1.3 Pa (10⁻² mm Hg) hergestellt. Hierzu wird zunächst eine 0,1 M Polydichlorphosphazenlösung unter Inertgasatmosphäre hergestellt (0,174 g auf 5 ml Lösungsmittel). Als Lösungsmittel wird absolutes Toluol verwendet. In diese Lösung wird dann 2,2,2-Natriumfluoroethanoat in absolutem Tetrahydrofuran als Lösungsmittel (8 ml absolutes Tetrahydrofuran, 0,23 g Natrium, 1,46 ml 2,2,2-Trifluorethanol) verestert.

B: Zur oxidativen Reinigung und gleichzeitigen Hydroxylierung der künstlichen Implantatoberflächen wird das Substrat in eine Mischung aus 30%iger H₂O₂ und konzentrierter Schwefelsäure (Carosche Säure) im Verhältnis 1 : 3 für 2 Stunden bei einer Reaktionstemperatur von 80° C gelegt. Nach dieser Behandlung wird das Substrat mit 0,5 l entmineralisiertem Wasser von 18 MOhmcm und etwa pH 5 gewaschen und anschließend im Stickstoffstrom getrocknet.

C: Zur Beschichtung der Implantatoberfläche mit einem Adhäsionspromotor wird das gemäß Beispiel 1.B mit Caroscher Säure oxidativ gereinigte künstliche Implantat in eine 2%ige Aminopropyltrimethoxysilanlösung in absolutem Ethanol für 30 Minuten bei Raumtemperatur eingetaucht. Daraufhin wird das Substrat mit 4-5 ml absolutem Ethanol gewaschen und 1 Stunde bei 105 °C im Trockenschrank belassen.

### Beispiel 2

A: Ein gemäß Beispiel 1B und 1C vorbehandeltes künstliches Implantat wurde 24 Stunden bei Raumtemperatur in eine 0.1 M Poly[bis(trifluoroethoxy)phosphazen]-Lösung in Ethylacetat (0,121 g auf 5 ml Ethylacetat), die 0,0121 g Probucol enthielt, eingebracht. Anschließend wurde das so hergestellte künstliche Implantat mit 4-5 ml Ethylacetat gewaschen und im Stickstoffstrom getrocknet.

B: Ein gemäß Beispiel 1B und 1C vorbehandeltes künstliches Implantat wurde 24 Stunden bei Raumtemperatur in eine 0.1 M Poly[bis(trifluoroethoxy)phosphazen]-Lösung in Ethylacetat (0,121 g auf 5 ml Ethylacetat), die 0,0242 g Trapidil enthielt, eingebracht. Anschließend wurde das so hergestellte künstliche Implantat mit 4-5 ml Ethylacetat gewaschen und im Stickstoffstrom getrocknet.

Die Oberfläche der in Beispielen 2A und 2B erhaltenen künstlichen Implantate wurde mittels der Photoelektronenspektroskopie auf die elementare Zusammensetzung, Stöchiometrie und Übetzugsdicke untersucht. Die Ergebnisse zeigten, dass die Immobilisierung des Poly[bis(trifluoroethoxy)phosphazens] über das Aminopropyltrimethoxysilan als Adhäsionspromotor erfolgte und Überzugsdicken von über 2,4 nm erreicht wurden. Weiterhin konnte analytisch gezeigt werden (NMR), dass Tapidil bzw. Probucol in entsprechender Menge in den Überzug eingelagert wurde.

### Beispiel 3

Ein gemäß Beispiel 1B gereinigtes künstliches Implantat wurde bei 70 °C in eine 0,1 M Poly[bis(trifluoroethoxy)phosphazen]lösung in Ethylacetat (0,121 g auf 5 ml Ethylacetat), die 0,0121 g Probucol enthielt, für 24 Stunden gelegt. Anschließend wurde das so behandelte künstliche Implantat mit 4 - 5 ml Ethylacetat gewaschen und im Stickstoffstrom getrocknet.

Das so hergestellte künstliche Implantat wurde mit Hilfe der Photoelektronenspektroskopie auf die elementare Zusammensetzung, Stöchiometrie und Überzugsdicke untersucht Die Ergebnisse zeigen, daß die Ankopplung des Poly[bis(trifluoroethoxy)phosphazen] auf der Implantatoberfläche erfolgt ist und Schichtdicken von über 2,1 nm erreicht wurden. Weiterhin konnte gezeigt werden, dass das Probucol in entsprechender Menge in den Überzug eingelagert wurde.

### Beispiel 4

A: Ein gemäß Beispiel 1B und 1C vorbehandeltes künstliches Implantat wurde 24 Stunden bei Raumtemperatur in eine 0.1 M Poly[bis(trifluoroethoxy)phosphazen]-Lösung in Ethylacetat (0,121 g auf 5 ml Ethylacetat) eingebracht. Anschließend wurde das so hergestellte künstliche Implantat mit 4-5 ml Ethylacetat gewaschen und im Stickstoffstrom getrocknet.

B: Das gemäß Beispiel 4A erhaltene Substrat wurde 24 h bei RT in eine Lösung von Cerivastatin in Ethylacetat (0,0121 g Cerivastatin auf 5 ml Ethylacetat) getaucht. Nach Trocknung in Stickstoffstrom konnte analytisch gezeigt werden, dass die Poly[bis(trifluoroethoxy)]phosphazen-Schicht Cerivastatin enthielt.

## Patentansprüche

1. Künstliches Implantat, umfassend ein Implantatmaterial als Substrat und einen mindestens teilweise auf die Oberfläche des Substrats aufgebrachten, biokompatiblen Überzug, der ein antithrombogenes Polymer mit der folgenden allgemeinen Formel (I) 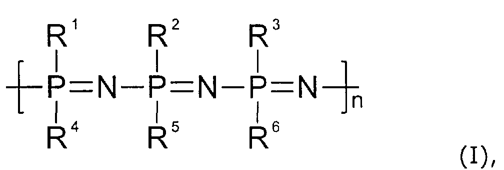 wobei R¹ bis R⁶ gleich oder unterschiedlich sind und einen Alkoxy-, Alkylsulfonyl-, Dialkylamino- oder Aryloxyrest oder einen Heterocycloalkyl- oder Heteroarylrest mit Stickstoff als Heteroatom bedeutet, und wenigstens einen weiteren pharmakologisch aktiven Wirkstoff umfasst.

2. Künstliches Implantat nach Anspruch 1, wobei mindestens einer der Reste R¹ bis R⁶ ein Alkoxyrest ist, der mit mindestens einem Fluoratom substituiert ist.

3. Künstliches Implantat nach Anspruch 2, wobei das antithrombogene Polymer Poly[bis(trifluoroethoxy)phosphazen] ist.

4. Künstliches Implantat nach einem der Ansprüche 1 bis 3, wobei der Wirkstoff ein antimitogener Wirkstoff, insbesondere ein Zytostatikum, ein PDGF-Antagonist, insbesondere Trapidil, oder ein Raf-1-Kinase-Hemmer ist.

5. Künstliches Implantat nach einem der Ansprüche 1 bis 3, wobei der Wirkstoff ein Radikalfänger (insbesondere Probucol), ein Antisensewirkstoff (insbesondere Plasmid-DNA), ein Statin (insbesondere Cerivastatin) oder ein GP-IIb/IIIa-Rezeptorantagonist (insbesondere Abciximab) ist.

6. Künstliches Implantat nach einem der Ansprüche 1 bis 5, wobei das Verhältnis von antithrombogenen Polymer zu Wirkstoff von 1:0,0001 bis 1:1, vorzugsweise von 1:0,05 bis 1:0,5 beträgt.

7. Künstliches Implantat nach einem der Ansprüche 1 bis 6, wobei zwischen der Oberfläche des Substrats und dem biokompatiblen Überzug eine Schicht angeordnet ist, die einen Adhäsionspromotor enthält.

8. Künstliches Implantat nach Anspruch 7, wobei der Adhäsionspromotor eine Silicium-organische Verbindung, insbesondere Aminopropyltrimethoxysilan ist.

9. Verfahren zur Herstellung eines künstlichen Implantats nach einem der Ansprüche 1 bis 8, wobei der biokompatible Überzug durch Umsetzen des Substrats mit
(a) einem Gemisches aus dem antithrombogenen Polymer oder eine Vorstufe desselben und des Wirkstoffs oder
(b) dem antithrombogenen Polymer oder einer Vorstufe desselben unter Erhalt eines primären Polymerüberzugs und nachfolgendes Aufbringen/Eindringen des Wirkstoffs in den primären Polymerüberzug
auf das Substrat aufgebracht wird.

10. Verfahren nach Anspruch 9, worin der Überzug nasschemisch, insbesondere durch Umsetzen des Substrats mit einem Gemisch aus antithrombogenem Polymer und Wirkstoff aufgebracht wird.

11. Verfahren nach Anspruch 10, worin das Lösungsmittel für das nasschemische Auftragen ausgewählt ist aus dipolar aprotischen Lösungsmitteln und vorzugsweise Ethylacetat ist.

12. Verfahren nach Anspruch 9 bis 11, worin vor dem Auftragen des biokompatiblen Überzugs ein Adhäsionspromotor auf die Oberfläche des Substrat aufgebracht wird.
